# EUROPÄISCHE PATENTANMELDUNG

(11) **EP 4 671 851 A1**
(43) Veröffentlichungstag der Anmeldung: **31.12.2025**
(21) Anmeldenummer: 24184002.4
(22) Anmeldetag: 24.06.2024
(51) Int. Cl.: G02C 3/00, A61F 9/02, G02C 11/02

(54) **BRILLE UMFASSEND FLAUSCHMATERIAL**

(71) Anmelder: IQ Brand, Design & Production GmbH, 6262 Schlitters (AT)
(72) Erfinder: EGGER, Christoph, 6262 Schlitters (AT); EGGER, Maximilian, 6262 Schlitters (AT)
(74) Vertreter: Weickmann & Weickmann PartmbB

(57) **Zusammenfassung**

Die Erfindung betrifft eine Brille, umfassend mindestens eine Durchsichtscheibe, mindestens eine Halterung, mindestens ein Halteband, und Befestigungsmittel, wobei das Halteband zumindest teilweise ein Flauschmaterial umfasst; ein Kit umfassend die Brille und dessen Verwendung.

## Beschreibung

Die Erfindung betrifft eine Brille, umfassend mindestens eine Durchsichtscheibe, mindestens eine Halterung, mindestens ein Halteband, und Befestigungsmittel, wobei das Halteband zumindest teilweise ein Flauschmaterial umfasst; ein Kit umfassend die Brille; und dessen Verwendung.

Brillen haben im Freizeitbereich und in der Arbeitssicherheit nicht nur als Sehhilfe, sondern auch als Schutzausrüstung eine große Bedeutung.

Sowohl bei der Arbeit als auch in der Freizeit, z.B. beim Sport, werden häufig Gegenstände benötigt, die möglichst leicht greifbar oder frei zugänglich sein und ggf. sichtbar getragen werden müssen. Solche Gegenstände können z.B. Werkzeuge, Zugangskarten, Namensschilder, Totmanngeräte, Lawinenpiepser, Sicherheitschips, medizinische Chips sowie Transponder, Sensoren, Sender, o.Ä. sein. Üblicherweise werden die Gegenstände in Taschen in der Bekleidung oder am Gürtel verstaut, wodurch die Gegenstände visuell verdeckt oder elektromagnetisch abgeschirmt werden, was deren Funktionsweise beeinträchtigt oder gar verhindert.

DE 3616820 A1 beschreibt eine Brille mit Brillenband, wobei das Brillenband mit zueinander komplementären Beschichtungen einer Klettverbindung versehen ist und die beiden Beschichtungen der Verbindung in einem gewissen Abstand voneinander angeordnet sind. Durch Umschlagen des Brillenbandes und Inkontaktbringen der Beschichtungen, lässt sich die Bandlänge variabel einstellen.

Eine Fixierung von Gegenständen an der Brille ist nicht vorgesehen.

Der Erfindung liegt die Aufgabe zu Grunde, eine Brille bereitzustellen, welche eine individuelle, stabile, sichtbare und schnelle Fixierung von Gegenständen ermöglicht.

Zur Lösung dieser Aufgabe wird erfindungsgemäß eine Brille bereitgestellt, umfassend
(i) mindestens eine Durchsichtscheibe,
(ii) mindestens eine Halterung,
(iii) mindestens ein Halteband, und
(iv) Befestigungsmittel, die eingerichtet sind, um das Halteband an mindestens einer Halterung zu befestigen,
wobei das Halteband zumindest teilweise ein Flauschmaterial umfasst.

Bevorzugt umfasst die erfindungsgemäße Brille
(i) mindestens eine Durchsichtscheibe,
(ii) eine Halterung,
(iii) mindestens ein Halteband, und
(iv) Befestigungsmittel, die eingerichtet sind, um das Halteband an einer Halterung zu befestigen,
wobei das Halteband zumindest teilweise ein Flauschmaterial umfasst.

In einer bevorzugten Ausführungsform der Erfindung, ist die Brille eine Skibrille, Motorradbrille, Motocross-Brille, Arbeitssicherheitsbrille, Fahrradbrille, oder taktische Brille.

Die Durchsichtscheibe ist bevorzugt transparent, polarisiert, getönt, entspiegelt und/oder verspiegelt.

Bevorzugt umfasst die Brille eine Durchsichtscheibe oder zwei Durchsichtscheiben, die übereinander in einem vorbestimmten Abstand angeordnet sind. Die Durchsichtscheiben können teilweise perforiert sein.

Die erste und die zweite Durchsichtscheibe sind bevorzugt hergestellt aus mindestens einem Thermoplast, insbesondere Polycarbonat oder thermoplastisches Polyurethan, Nylon, Duroplast, wie z.B. Polyurethan, oder Glas, besonders bevorzugt Polycarbonat, Polyurethan oder Glas.

Die Durchsichtscheibe umfasst bevorzugt eine Aussparung. Die Aussparung der Durchsichtscheibe ist so geformt, dass sie ein Aufsetzen der Brille auf die Nase ermöglicht und trägt zu einer stabilen Sitzfestigkeit der Brille bei.

Die Halterung nimmt bevorzugt mindestens eine Durchsichtscheibe zumindest teilweise auf. Bevorzugt nimmt die Halterung die Durchsichtscheibe(n) vollständig auf. Dies bietet optimalen Schutz der Gläser, eine stabile Struktur der Brille, und erlaubt eine gute Abdichtung gegenüber Staub, Insekten, etc.

Die Halterung bildet bevorzugt einen Brillenrahmen.

Die Halterung weist bevorzugt mindestens ein Verbindungselement, z.B. eine Öse, einen Schnappverschluss, Haken, oder Stift auf. Das Verbindungselement ermöglicht eine Befestigung oder Fixierung von z.B. einem Halteband, einer Telekommunikationsvorrichtung, etc., insbesondere von einem Halteband.

Die Halterung ist bevorzugt aus einem Thermoplast, z.B. Polyolefin, Polyamid, thermoplastisches Polyurethan, oder Polycarbonat, oder Duroplast, wie z.B. Polyurethan oder Gummi, gebildet.

Die Halterung weist bevorzugt eine proximal liegende aus Schaumstoff gebildete Randeinfassung auf. Die Polsterung des Schaumstoffs zwischen der Halterung und dem Träger erhöht die Sitzfestigkeit der Brille und vermindert den auf den Träger ausgeübten Druck, was das Tragen der Brille über einen langen Zeitraum ohne Einschränkung des Tragekomforts ermöglicht. Der Schaumstoff ist bevorzugt ein Polyurethan oder geschäumtes Polyolefin.

Die erfindungsgemäße Brille umfasst ein Halteband. Das Halteband ist bevorzugt elastisch und weist insbesondere eine Dehnbarkeit von 20-300 %, stärker bevorzugt, 50-250 % bezogen auf den ungedehnten Zustand auf. Dadurch wird eine sitzfeste, flexible und bequeme Passform für diverse Kopfgrößen ermöglicht. Das Halteband lässt sich unter Zugbelastung mechanisch dehnen und kehrt bei Entlastung zumindest weitgehend in seine Ursprungsform zurück.

Das Halteband umfasst bevorzugt Gummi, Elasthan und/oder Textil.

Das Halteband ist bevorzugt längenverstellbar. So kann eine flexible und individuelle Anpassung an Passform und Kopfgröße gewährleistet werden. Die Brille umfasst daher ferner bevorzugt Einstellmittel, z.B. Klettverschluss oder Laschen/Schlaufen-Systeme, welche eingerichtet sind, um die Passform oder Länge des Haltebands einzustellen.

Das Halteband hat bevorzugt eine Breite von 0,5-10, insbesondere 0,5-5 cm.

Das Halteband umfasst zumindest teilweise ein Flauschmaterial. Das Flauschmaterial ist bevorzugt auf der distalen Seite des Haltebandes angebracht.

Das Flauschmaterial erstreckt sich bevorzugt entlang der Längsseite, insbesondere der distalen Längsseite, des Haltebandes kontinuierlich. Das Flauschmaterial erstreckt sich bevorzugt entlang der Längsseite, insbesondere der distalen Längsseite, über eine Länge von mindestens 10 cm, im ungedehnten Zustand.

Das Flauschmaterial erstreckt sich bevorzugt entlang der distalen Querseite zumindest teilweise, insbesondere kontinuierlich. Das Flauschmaterial erstreckt sich bevorzugt entlang der Querseite, insbesondere der distalen Querseite, über eine Länge von 0,5-10 cm, stärker bevorzugt 0,5-9,5 cm, im ungedehnten Zustand.

In einer bevorzugten Ausführungsform der Erfindung, umfasst das Halteband ein elastisches Trägerband, auf das das Flauschmaterial aufgebracht ist. Das Trägerband ist bevorzugt aus Gummi, Polyurethan oder Elasthan.

Das Flauschmaterial bedeckt bevorzugt das elastische Trägerband zumindest teilweise.

In einer bevorzugten Ausführungsform der Erfindung, ist das Flauschmaterial auf das Trägerband geschweißt, geklebt, genäht, gesteppt, oder gewebt.

Das Flauschmaterial ist bevorzugt am Längsrand des Trägerbandes verschweißt, gesteppt, silikonisiert, oder geklebt. Diese Ausführungsform verhindert ein Ausfransen des Flauschmaterials an den Längsseiten.

In einer weiteren bevorzugten Ausführungsform der Erfindung, ist das Flauschmaterial eingerichtet für eine Klettverbindung.

Das Flauschmaterial ist bevorzugt ein Vlies, das bevorzugt aus Kunststofffasern, z.B. Nylon-, Polyester-, Polypropylen-, Viskose-, Cellulose(derivat)- oder Mischgewebe-Fasern gebildet ist. Die Kunststofffaser ist so ausgewählt, dass sie eine der Benutzung der Brille entsprechenden Weichheit, Festigkeit, Haltbarkeit, Abriebfestigkeit und Hautverträglichkeit aufweist.

In einer bevorzugten Ausführungsform der Erfindung, umfasst das Flauschmaterial nicht lösbare Schlaufen oder Pilzköpfe.

Die nicht lösbaren Schlaufen oder Pilzköpfe weisen bevorzugt eine Länge von 2-10 mm auf, insbesondere 3-10 mm. Die nicht lösbaren Schlaufen oder Pilzköpfe weisen bevorzugt eine Dichte von 100-300 Schlaufen/cm² oder 20-100 Pilzköpfen/cm² auf. Die nicht lösbaren Schlaufen weisen bevorzugt einen maximalen Durchmesser von 2-10 mm auf. Die Kombination aus Länge, Dichte und des Durchmessers von Schlaufen und Pilzköpfen ist so gewählt, dass Sie einen bezüglich der Anwendung optimalen Kompromiss zwischen Haftfestigkeit und Komfort der Handhabung darstellt. Die nicht lösbaren Schlaufen oder Pilzköpfe können bevorzugt reflektierende Eigenschaften aufweisen, um die Sichtbarkeit bei schlechten Sichtverhältnissen oder Dunkelheit zu erhöhen.

Die nicht lösbaren Schlaufen oder Pilzköpfe weisen insbesondere eine Reißfestigkeit von mindestens 100-500 N/cm auf, insbesondere 100-300 N/cm gemessen gemäß DIN EN ISO 527.

Die Brille umfasst Befestigungsmittel, die eingerichtet sind, um das Halteband an mindestens einer Halterung zu befestigen. Das Befestigungsmittel ist bevorzugt als Nietverbindung, Klebeverbindung, Haltestift, Schnappverschluss oder Nahtverbindung eingerichtet.

Ein weiterer Aspekt der Erfindung betrifft ein Kit umfassend
- die erfindungsgemäße Brille und
- mindestens einen Gegenstand, der mit Widerhacken oder Pilzköpfen versehen ist, die für eine Klettverbindung eingerichtet sind.

Der Gegenstand umfasst bevorzugt ein Leuchtmittel, Sender, Patch, Transponder, wie z.B. RFID, NFC, Bluetooth-Einrichtung, UWB-Einrichtung, Haltevorrichtung, Sensor oder Kamera.

Der Gegenstand ist bevorzugt ein Lawinenpiepser, Totmanngerät, Sensor, medizinischer Chip, Skiticket, eine Zugangskarte oder Kamera. Bei diesen Gegenständen kommt es insbesondere darauf an, dass eine leichte Greifbarkeit oder freie Zugänglichkeit, Sichtbarkeit und keine elektromagnetische und/oder visuelle Abschirmung gewährleistet wird. Beispielsweise müssen Rettungskräfte nicht nur erkennen können, dass die zu rettende Person einen medizinischen Chip trägt, sondern müssen den Chip auch möglichst schnell und zuverlässig auslesen können. In diesem Fall ist eine freie Zugänglichkeit (sowohl visuell als auch elektromagnetisch) lebenserhaltend.

Die mit Widerhaken oder Pilzköpfen versehene Fläche des Gegenstands ist bevorzugt im Bereich von 1 cm x 1cm - 20 cm x 20 cm.

Ein weiterer Aspekt der Erfindung betrifft die Verwendung des Kits, wobei der Gegenstand mittels Widerhacken oder Pilzköpfen an dem Halteband fixiert wird. Unterschiedliche Gegenstände können so am Flauschmaterial des Haltebands befestigt werden. Alternativ kann auch ein Gegenstand über mehrere Berührpunkte am Flauschmaterial befestigt werden. Dadurch können auch große und/oder schwere Gegenstände z.B. dreidimensional stabil fixiert werden. Dies ist z.B. bei Kamerahalterungen, Stativen oder Kameras von großer Bedeutung.

Ein Ausführungsbeispiel der Erfindung wird nachstehend unter Bezugnahme auf die Figuren näher erläutert.
**Fig. 1** zeigt in Vorderansicht eine Brille gemäß der vorliegenden Erfindung
**Fig. 2** zeigt mögliche Ausführungsformen des Haltebands
**Fig. 3** zeigt mögliche Ausführungsformen der Widerhaken und Pilzköpfe des Gegenstands.

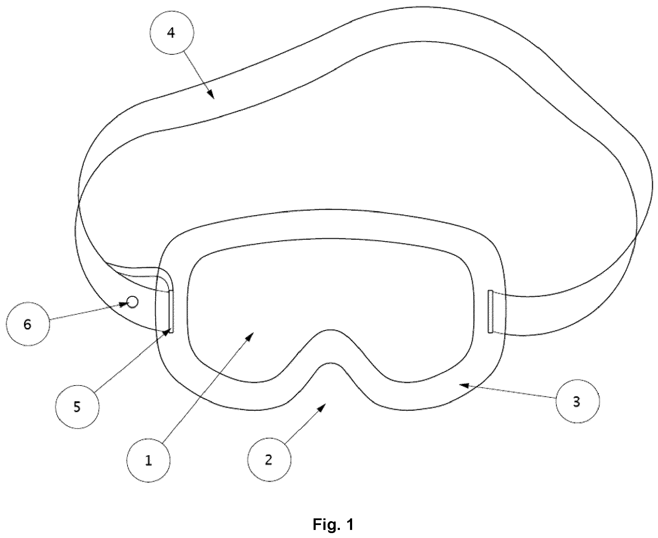

**Fig. 1** zeigt in Vorderansicht eine Halterung (3), die eine Aussparung (2) für die Nase aufweist. Die Halterung (3) nimmt die Durchsichtscheibe (1) vollständig auf und umfasst ein Verbindungselement (6) in Form einer Öse (5). Ein Halteband (4) ist an der Halterung (3) durch die Öse (5) mittels Befestigungsmittel (6) befestigt.

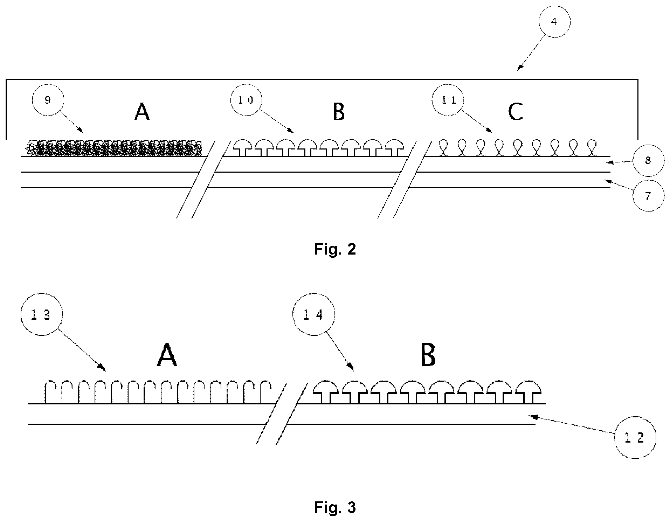

**Fig. 2** zeigt mögliche Ausführungsformen des Haltebands (4) in Schnittdarstellung. Das Halteband (4) umfasst ein Trägerband (7), auf das ein Flauschmaterial (8) angeordnet ist. Das Flauschmaterial (8) kann A: Vlies (9), B: Pilzköpfe (10) oder C: Loops (11) umfassen.

**Fig. 3** zeigt mögliche Ausführungsformen der Widerhaken und Pilzköpfe des Gegenstands (12) in Schnittdarstellung (A: Widerhaken (13); B: Pilzköpfe (14)).

Die vorliegende Erfindung umfasst folgende Punkte:
1. Brille, umfassend
   (i) mindestens eine Durchsichtscheibe,
   (ii) mindestens eine Halterung,
   (iii) mindestens ein Halteband, und
   (iv) Befestigungsmittel, die eingerichtet sind, um das Halteband an mindestens einer Halterung zu befestigen,
   wobei das Halteband zumindest teilweise ein Flauschmaterial umfasst.
2. Brille nach Anspruch 1, wobei die Brille eine Skibrille, Motorradbrille, Motocross-Brille, Arbeitssicherheitsbrille, Fahrradbrille, oder taktische Brille ist.
3. Brille nach Punkt 1 oder 2, wobei die Durchsichtscheibe transparent, polarisiert, getönt, entspiegelt, polarisiert und/oder verspiegelt ist.
4. Brille nach einem der vorhergehenden Punkte, wobei die Durchsichtscheibe hergestellt ist aus mindestens einem Thermoplast, insbesondere Polycarbonat, Polyurethan oder Nylon, oder Glas.
5. Brille nach einem der vorhergehenden Punkte, wobei die Durchsichtscheibe eine Aussparung umfasst.
6. Brille nach einem der vorhergehenden Punkte, wobei die Halterung mindestens eine Durchsichtscheibe zumindest teilweise aufnimmt.
7. Brille nach einem der vorhergehenden Punkte, wobei die Halterung ein Brillenrahmen ist.
8. Brille nach einem der vorhergehenden Punkte, wobei die Halterung mindestens ein Verbindungselement, z.B. eine Öse, ein Schnappverschluss, Haken, oder Stift aufweist.
9. Brille nach einem der vorhergehenden Punkte, wobei die Halterung aus einem Thermoplast, z.B. Polyolefin, Polyamid, thermoplastischem Polyurethan, oder Polycarbonat, oder Duroplast, wie z.B. Polyurethan oder Gummi, gebildet ist,
10. Brille nach einem der vorhergehenden Punkte, wobei die Halterung eine proximal liegende aus Schaumstoff gebildete Randeinfassung aufweist.
11. Brille nach einem der vorhergehenden Punkte, wobei das Halteband elastisch ist und insbesondere eine Dehnbarkeit von 20-300 %, stärker bevorzugt, 50-250 % aufweist.
12. Brille nach Punkt 11, wobei das Halteband Gummi, Elasthan und/oder Textil umfasst.
13. Brille nach einem der vorgehenden Punkte, wobei das Halteband längenverstellbar ist.
14. Brille nach einem der vorhergehenden Punkte, ferner umfassend Einstellmittel, z.B. Klettverschluss oder Laschen/Schlaufen-System, welche eingerichtet sind, um die Passform oder Länge des Haltebands einzustellen.
15. Brille nach einem der vorhergehenden Punkte, wobei das Halteband eine Breite von 0,5-10, insbesondere 0,5-5 cm, hat.
16. Brille nach einem der vorhergehenden Punkte, wobei das Flauschmaterial auf der distalen Seite des Haltebandes angebracht ist.
17. Brille nach einem der vorhergehenden Punkte, wobei sich das Flauschmaterial entlang der distalen Längsseite des Haltebandes kontinuierlich erstreckt.
18. Brille nach einem der vorhergehenden Punkte, wobei sich das Flauschmaterial entlang der distalen Querseite zumindest teilweise erstreckt, insbesondere kontinuierlich erstreckt.
19. Brille nach einem der vorgehenden Punkte, wobei das Halteband ein elastisches Trägerband umfasst, auf das das Flauschmaterial aufgebracht ist.
20. Brille nach Punkt 19, wobei das Flauschmaterial das elastische Trägerband zumindest teilweise bedeckt.
21. Brille nach einem der Punkte 19 oder 20, wobei das Flauschmaterial auf das Trägerband geschweißt, geklebt, genäht, gesteppt, oder gewebt ist.
22. Brille nach einem der vorhergehenden Punkte 19-21, wobei das Flauschmaterial am Längsrand des Trägerbandes verschweißt, gesteppt, silikonisiert, oder geklebt ist.
23. Brille nach einem der vorhergehenden Punkte, wobei das Flauschmaterial eingerichtet ist für eine Klettverbindung.
24. Brille nach einem der vorhergehenden Punkte, wobei das Flauschmaterial aus Kunststofffasern gebildet ist.
25. Brille nach einem der vorhergehenden Punkte, wobei das Flauschmaterial nicht lösbare Schlaufen oder Pilzköpfe umfasst.
26. Brille nach einem der vorhergehenden Punkte, wobei das Befestigungsmittel als Nietverbindung, Klebeverbindung oder Nahtverbindung eingerichtet ist.
27. Kit umfassend
   - eine Brille nach einem der Punkte 1-26 und
   - mindestens einen Gegenstand, der mit Widerhaken oder Pilzköpfen versehen ist, die für eine Klettverbindung eingerichtet sind.
28. Kit nach Anspruch 27, wobei der Gegenstand Leuchtmittel, Sender, Patch, Transponder, wie z.B. RFID, NFC, Bluetooth-Einrichtung, UWB-Einrichtung, Haltevorrichtung, Sensor oder Kamera umfasst.
29. Kit nach Anspruch 27 oder 28, wobei der Gegenstand ein Lawinenpiepser, Totmanngerät, Sensor, medizinischer Chip, eine Zugangskarte, Kamera oder Skiticket ist.
30. Kit nach einem der Punkte 27-29, wobei die mit Widerhaken oder Pilzköpfen versehene Fläche des Gegenstands im Bereich von 1 cm x 1cm - 20 cm x 20 cm ist.
31. Verwendung eines Kits nach einem der Punkte 27-30, wobei der Gegenstand mittels Widerhacken oder Pilzköpfen an dem Halteband fixiert wird.

## Patentansprüche

1. Brille, insbesondere Skibrille, Motorradbrille, Motocross-Brille, Arbeitssicherheitsbrille, Fahrradbrille, oder taktische Brille, umfassend
(i) mindestens eine Durchsichtscheibe,
(ii) mindestens eine Halterung,
(iii) mindestens ein Halteband, und
(iv) Befestigungsmittel, die eingerichtet sind, um das Halteband an mindestens einer Halterung zu befestigen,
wobei das Halteband zumindest teilweise ein Flauschmaterial umfasst.

2. Brille nach einem der vorhergehenden Ansprüche, wobei die Halterung, insbesondere ein Brillenrahmen, mindestens eine Durchsichtscheibe zumindest teilweise aufnimmt und/oder mindestens ein Verbindungselement, z.B. eine Öse, ein Schnappverschluss, Haken, oder Stift aufweist und/oder eine proximal liegende aus Schaumstoff gebildete Randeinfassung aufweist.

3. Brille nach einem der vorhergehenden Ansprüche, wobei das Halteband elastisch ist und insbesondere eine Dehnbarkeit von 20-300 %, stärker bevorzugt, 50-250 % aufweist und/oder Gummi, Elasthan und/oder Textil umfasst und/oder längenverstellbar ist.

4. Brille nach einem der vorhergehenden Ansprüche, ferner umfassend Einstellmittel, z.B. Klettverschluss oder Laschen/Schlaufen-System, welche eingerichtet sind, um die Passform oder Länge des Haltebands einzustellen.

5. Brille nach einem der vorhergehenden Ansprüche, wobei das Flauschmaterial auf der distalen Seite des Haltebandes angebracht ist.

6. Brille nach einem der vorhergehenden Ansprüche, wobei sich das Flauschmaterial entlang der distalen Längsseite des Haltebandes kontinuierlich erstreckt und/oder wobei sich das Flauschmaterial entlang der distalen Querseite zumindest teilweise erstreckt, insbesondere kontinuierlich erstreckt.

7. Brille nach einem der vorgehenden Ansprüche, wobei das Halteband ein elastisches Trägerband umfasst, auf das das Flauschmaterial aufgebracht ist und/oder das Flauschmaterial das elastische Trägerband zumindest teilweise bedeckt.

8. Brille nach Anspruch 7, wobei das Flauschmaterial auf das Trägerband geschweißt, geklebt, genäht, gesteppt, oder gewebt ist und/oder das Flauschmaterial am Längsrand des Trägerbandes verschweißt, gesteppt, silikonisiert, oder geklebt ist.

9. Brille nach einem der vorhergehenden Ansprüche, wobei das Flauschmaterial eingerichtet ist für eine Klettverbindung.

10. Brille nach einem der vorhergehenden Ansprüche, wobei das Flauschmaterial aus Kunststofffasern gebildet ist.

11. Brille nach einem der vorhergehenden Ansprüche, wobei das Flauschmaterial nicht lösbare Schlaufen oder Pilzköpfe umfasst.

12. Brille nach einem der vorhergehenden Ansprüche, wobei das Befestigungsmittel als Nietverbindung, Klebeverbindung oder Nahtverbindung eingerichtet ist.

13. Kit umfassend
- eine Brille nach einem der Ansprüche 1-12 und
- mindestens einen Gegenstand, der mit Widerhaken oder Pilzköpfen versehen ist, die für eine Klettverbindung eingerichtet sind, wobei der Gegenstand bevorzugt Leuchtmittel, Sender, Patch, Transponder, wie z.B. RFID, NFC, Bluetooth-Einrichtung, UWB-Einrichtung, Haltevorrichtung, Sensor oder Kamera umfasst.

14. Kit nach Anspruch 13, wobei der Gegenstand ein Lawinenpiepser, Totmanngerät, Sensor, medizinischer Chip, eine Zugangskarte, Kamera oder Skiticket ist.

15. Verwendung eines Kits nach einem der Ansprüche 13-14, wobei der Gegenstand mittels Widerhacken oder Pilzköpfen an dem Halteband fixiert wird.
